# EUROPÄISCHE PATENTSCHRIFT

(11) **EP 2 948 083 B1**
(45) Veröffentlichungstag und Bekanntmachung des Hinweises auf die Patenterteilung: **16.08.2017**
(21) Anmeldenummer: 14711483.9
(22) Anmeldetag: 17.03.2014
(51) Int. Cl.: A61B 17/86

(54) **CHIRURGISCHE ARRETIERSCHRAUBE**
SURGICAL LOCKING SCREW
VIS D'ARRÊT CHIRURGICALE

(30) Priorität: 15.03.2013 DE 202013101135 U
(43) Veröffentlichungstag der Anmeldung: 02.12.2015
(73) Patentinhaber: Zimmer GmbH, 8404 Winterthur (CH)
(72) Erfinder: MILDNER, Alexander, 1080 Wien (AT); KOTULJAC, Vladko, 72355 Schömberg (DE)
(74) Vertreter: Manitz Finsterwald Patentanwälte PartmbB
(86) Internationale Anmeldenummer: PCT/EP2014/055256
(87) Internationale Veröffentlichungsnummer: WO 2014/140360

(56) Entgegenhaltungen:
- EP-A1- 1 033 111
- EP-A2- 1 870 050
- US-A1- 2010 094 352
- US-B1- 6 565 566

## Beschreibung

Die Erfindung betrifft eine chirurgische Arretierschraube gemäß dem Oberbegriff des Anspruchs 1 zum Fixieren von Knochenteilen bei einem Fersenbruch, insbesondere von Knochentrümmern bei einem Fersentrümmerbruch. Die Arretierschraube besteht beispielsweise aus einem metallischen Werkstoff, kann aber ebenso aus einem geeigneten nichtmetallischen Werkstoff bestehen oder einen solchen umfassen.

US 2010/094352 A1, EP 1 033 111 A1 sowie US 6 565 566 B1 offenbaren jeweils eine chirurgische Arretierschraube gemäß dem Oberbegriff des Anspruchs 1.

Um eine gute Heilung von Fersenbrüchen zu gewährleisten, ist es notwendig/sinnvoll, die Knochenstücke relativ zueinander zu fixieren, bevor der gesamte Fuß eingegipst wird.

Der Erfindung liegt die Aufgabe zugrunde, für diesen Zweck geeignete Arretier- bzw. Positioniermittel anzugeben.

Diese Aufgabe wird mit einer chirurgischen Arretierschraube mit den Merkmalen des Anspruchs 1 gelöst
Der Erfindung liegt der Gedanke zugrunde, eine Arretierschraube zum Fixieren von Knochenteilen bei einem Fersenbruch zur Verfügung zu stellen,welche es ermöglicht, mindestens eine Arretierschraube derart in die gebrochene Ferse einzuschrauben, dass die Arretierschraube möglichst viele Knochenteile unmittelbar kontaktiert bzw. durchsetzt. Um eine rotationsstabile Fixierung der Knochenteile zu ermöglichen, hat es sich bewährt, zwei derartige Arretierschrauben einzusetzen, die zumindest teilweise voneinander beabstandet sind.

Die hier beschriebene Arretierschraube zeichnet sich dadurch aus, dass diese ein zumindest näherungsweise axial durchgehendes, d. h. nicht unterbrochenes Außengewinde aufweist, welches durchgehend eine gleichbleibende Gewindesteigung aufweist, um eine Kompression der Knochenteile zu verhindern. Es hat sich überraschend herausgestellt, dass hierdurch die Heilung nachhaltig verbessert wird. In diesem Zusammenhang bezieht sich die konstante Gewindesteigung auf einen Gewindegang. Das heißt, bei einem mehrgängigen Gewinde ist diese Steigung als der Abstand zweier Gewindespitzen ein und desselben Gewindegangs gegeben.

Um die Gefahr einer Kompression weiter zu minimieren, ist zusätzlich vorgesehen, dass ein Kerndurchmesser des Außengewindes sowohl in einem unten noch zu erläuternden ersten, distalen, Axialabschnitt als auch in einem in Einschraubrichtung dahinter, also proximal davon, befindlichen zweiten Axialabschnitt konstant ist, obwohl der Außendurchmesser des Außengewindes in dem zweiten (hinteren bzw. antriebsseitgen) Axialabschnitt mit zunehmendem Abstand zum vorderen Ende, das heißt von distal nach proximal, zunimmt. Diese konstruktive Maßnahme dient dazu, trotz eines Verzichtes auf eine Kompression eine definierte Lage der Arretierschraube im Fuß zu gewährleisten, und zwar dahin gehend, dass ein selbsttätiger Vortrieb in der Einschraubrichtung sicher verhindert wird, indem durch den steigenden Außengewindedurchmesser im zweiten Axialabschnitt die Reibung am Ende des Eindrehens in einen zylindrischen Bohrkanal erhöht wird. Gleichzeitig wird durch die erhöhte Reibung der weiter außen liegenden Gewindespitzen ein selbsttätiges Lösen der Schraube verhindert. Der Arretierschraubenkern weist hingegen eine axial durchgehende zylindrische Form auf, ggf. bis auf axial vordere und/oder hintere Anfasungen, die fakultativ vorgesehen sein können.

Mit anderen Worten zeichnet sich die erfindungsgemäße Arretierschraube trotz Verzichts auf unterschiedliche Gewindesteigungen und auf einen bei Kompressionsschrauben üblichen Durchmesseranstieg des Kerndurchmessers des Außengewindes durch eine Arretier- und Anschlagfunktion aus, die eine definierte Positionierung ermöglicht, wobei die Arretier- und Anschlagsfunktion bei in diesem Axialabschnitt gleichbleibendem Kerndurchmesser durch einen in Richtung Schraubenende ansteigenden Außengewindedurchmesser und eine dadurch vergrößerte Reibung des Gewindes im Knochen realisiert ist. Unter einer Anschlagfunktion im hier benutzten Sinne ist kein fester, formschlüssiger mechanischer Anschlag zu verstehen.

Vorteilhafte Ausbildungen der Erfindung sind den Unteransprüchen, der Beschreibung und der Zeichnung zu entnehmen.

Weiterbildungsgemäß ist vorgesehen, dass die Arretierschraube eine axial durchgehende, zentrische Kanülierung (Zentralkanal) zur Aufnahme eines Kirschnerdrahtes aufweist.

Insbesondere ist der Innendurchmesser der Kanülierung aus einem Wertebereich zwischen etwa 1,0 mm und 3,0 mm gewählt und beträgt in einer Ausführungsform zumindest näherungsweise 2,2 mm. Das weiterbildungsgemäße Vorsehen einer Kanülierung für einen Kirschnerdraht gewährleistet eine optimale, zielgerichtete Einbringbarkeit der chirurgischen Arretierschraube in den menschlichen Fuß.

Gemäß einer Ausführungsform kann das Außengewinde, wie unten erläutert wird, in einem vorderen Bereich als selbstschneidendes Gewinde ausgebildet sein und/oder in anderweitig ausgestalteten Einschneidmitteln ausmünden.

Alternativ oder zusätzlich kann das Außengewinde ferner zumindest bereichsweise einen Flankenwinkel aufweisen, der insbesondere zwischen 35° und 45° beträgt, und/oder das Außengewinde kann zumindest bereichsweise trapezförmig ausgebildet sein.

Im Falle des vorerwähnten Vorsehens einer axial durchgehenden Kanülierung ist der Kernabschnitt bzw. Kernbereich der Schraube hohlzylindrisch ausgebildet. Beispielsweise ist der Kerndurchmesser des Außengewindes - ob kanüliert oder nicht - aus einem Wertebereich zwischen 3,5 mm und 7 mm, insbesondere zwischen 4 mm und 6 mm gewählt. Insbesondere beträgt er zumindest näherungsweise 5 mm. Mit diesen Dimensionen ergeben sich eine gute Handhabbarkeit der Schraube, eine gute Einpassbarkeit in den Knochen bei geringer Schwächung des Knochenmaterials und eine hinreichend große Festigkeit der Schraube,

Als vorteilhaft hat es sich herausgestellt, wenn derjenige zweite Axialabschnitt, in dem der Außendurchmesser des Außengewindes mit zunehmendem Abstand zum vorderen Ende zunimmt, eine konische Hüllkontur mit einem Öffnungswinkel aufweist, der insbesondere als Kegelvollwinkel zwischen 5° und 6° beträgt.

Besonders gute Ergebnisse zur Erzielung einer reibungsbedingten Fixierfunktion bei gleichzeitiger Vermeidung einer Kompression werden weiterbildungsgemäß erreicht, wenn ein Außendurchmesserverhältnis eines maximalen Außendurchmessers des zweiten Axialabschnittes zu dem konstanten Außendurchmesser des ersten Axialabschnittes aus einem Wertebereich zwischen 1,0 und 1,4, insbesondere zwischen 1,1 und 1,3 gewählt ist. Insbesondere beträgt das Verhältnis zumindest näherungsweise 1,2. Mit diesen Verhältnissen resultiert eine gute reibungsbedingte Hemmung der Gewindegänge in der Endlage der implantierten Schraube.

Insbesondere kann der maximale Außendurchmesser des zweiten Axialabschnittes auch dem maximalen Außendurchmesser des Außengewindes insgesamt entsprechen. Insbesondere stellt der maximale Außendurchmesser des zweiten Axialabschnittes den maximalen Durchmesser der Schraube insgesamt dar, d.h. die Schraube umfasst keinen Teil oder Abschnitt, insbesondere keinen Kopf, dessen größter Durchmesser größer als der maximale Außendurchmesser des zweiten Axialabschnittes ist. Insofern kann in dieser Ausführungsform die Schraube als "Madenschraube" bezeichnet werden. Die Schraube weist demnach keinen formschlüssigen mechanischen Anschlag auf, der die Einschraubtiefe der Schraube fest begrenzen würde.

Der Außendurchmesser des Außengewindes in dem ersten Axialabschnitt ist aus einem Wertebereich zwischen 5,5 mm und 10 mm, insbesondere zwischen 6 mm und 8 mm, gewählt und beträgt insbesondere zumindest näherungsweise 7,5 mm. Weiter ist der maximale Außendurchmesser des Außengewindes im zweiten Axialabschnitt insbesondere aus einem Wertebereich zwischen 7 mm und 12 mm, insbesondere zwischen 8 mm und 11 mm gewählt und beträgt beispielsweise zumindest näherungsweise 9 mm. Mit diesen Dimensionen ergeben sich eine gute Handhabbarkeit der Schraube, eine gute Einpassbarkeit in den Knochen bei geringer Schwächung des Knochenmaterials, eine hinreichend große Festigkeit der Schraube, sowie eine gute reibungsbedingte Hemmung der Gewindegänge in der Endlage der implantierten Schraube.

Ebenso wirkt es sich positiv auf die Arretierfunktion bzw. die Gewährleistung einer definierten Einbringlage aus, wenn das Längenverhältnis der Gesamtlänge der Arretierschraube zur Länge des, insbesondere eine konische Hüllkontur aufweisenden, zweiten Axialabschnittes aus einem Wertebereich zwischen 2,5 und 5,0, insbesondere zwischen 3,0 und 4,5 gewählt ist.

Besonders zweckmäßig ist es dabei, wenn die Gesamtlänge der Arretierschraube aus einem Wertebereich zwischen 50 mm und 100 mm, insbesondere zwischen 60 mm und 90 mm gewählt ist und insbesondere 65 mm, 70 mm, 75 mm, 80 mm oder 85 mm beträgt. Die Länge des zweiten Axialabschnittes ist beispielsweise aus einem Wertebereich zwischen 10 mm und 30 mm gewählt und beträgt insbesondere zumindest näherungsweise 20 mm. Die Wahl diese Geometrieparameter vermag die Funktion der Schraube in einer Ausführungsform weiter zu unterstützen.

Erfindungsgemäß ist am axial hinteren, proximalen Ende der Schraube ein sich beispielsweise über ein bis zwei Gewindegänge erstreckender dritter Außengewindeabschnitt angeordnet, mit gegenüber dem größten Durchmesser des zweiten Axialabschnitts reduziertem Außengewindedurchmesser, wobei der Außengewindedurchmesser in Richtung des hinteren, proximalen, Endes der Schraube insbesondere abnimmt. Diese Verjüngung gewährleistet die Minimierung eines etwaigen Überstandes bzw. eine verbesserte Anpassung an anatomische Gegebenheiten, wodurch der Heilungserfolg verbessert wird. Selbstverständlich kann sich der zweite Axialabschnitt in einer Ausführungsform auch bis zum axial hinteren, proximalen, Ende der Schraube erstrecken.

Um einen sicheren Halt der Arretierschraube zu gewährleisten, kann vorgesehen sein, dass die Arretierschraube als selbstschneidende Schraube ausgebildet ist, sodass maximal eine Kerndurchmesserbohrung vorgebohrt werden muss und sich das Außengewinde der Arretierschraube selbsttätig in das Gewebe bzw. den Knochen einschraubt. Zu diesem Zweck sind weiterbildungsgemäß Schneidmittel vorgesehen, die insbesondere am vorderen Ende der Schraube angebracht sind, wobei die Schneidmittel zumindest eine sich von radial außen nach radial innen erstreckende Vertiefung umfassen können, die einen Gewindegang unterbricht.

In einer weiteren Ausführungsform der Arretierschraube ist eine axial vordere, distale, Stirnseite als Fräskopf ausgeführt. Beispielsweise sind eine oder mehrere, beispielsweise drei, sich radial erstreckende oder radial verlaufende und insbesondere axial nach vorne wirkende Schneiden an der distalen Stirnseite der Schraube angeordnet. Dies ermöglicht es, die Schraube selbstbohrend auszuführen, derart, dass sie direkt, d.h. ohne vorzubohren, eingesetzt werden kann. Dabei kann eine kanüllierte Schraube direkt über einen als Führung dienenden Kirschnerdraht implantiert werden.

Die hintere, proximale, Stirnseite der Schraube ist in einer Ausführungsform mit einem Antrieb und/oder einem Ansatz für ein Werkzeug versehen, beispielsweise einem Innensechskant oder einem Torx-Antrieb.

Das Außengewinde kann wenigstens einen mehrgängigen Abschnitt mit zwei oder mehr als zwei Gewindegängen aufweisen, wobei insbesondere jeder Gewindegang des mehrgängigen Abschnittes für sich genommen eine konstante Gewindesteigung aufweist. Ferner kann sich der mehrgängige Abschnitt zumindest über einen Bereich der Axialerstreckung des Außengewindes erstrecken, d. h. das Außengewinde muss nicht zwingend über seine gesamte axiale Länge mehrgängig ausgebildet sein. Dabei kann jeder Gewindegang des mehrgängigen Abschnittes eine konstante Gewindesteigung aufweisen.

Zusammenfassend zeichnet sich die hier beschriebene Arretierschraube unter anderem durch folgende Eigenschaften aus: Durch den zylindrischen Kern und die konstante Gewindesteigung wird eine unerwünschte zusätzliche Kompression auf den Frakturspalt vermieden. Der distale Gewindeabschnitt mit konstantem Außendurchmesser, der sich insbesondere über einen großen Teil der Länge der Arretierschraube erstreckt, ermöglicht eine einfache Implantation, da nur im Bereich des axial vordersten, distalen, Gewindeganges das Gewinde geschnitten werden muss, während der Rest dieses Gewindes mit konstantem Außendurchmesser durch die vorgeschnittenen Gewindegänge gleitet. Gegen Ende der Implantationsphase gelangt der Abschnitt mit dem sich von distal nach proximal vergrößernden Gewindeaußendurchmesser in Eingriff mit dem Knochen. Dieser Gewindeabschnitt muss sich mit weiterem axialem Vorschub stetig neu einschneiden. Dadurch liegen die Gewindespitzen dieses Abschnitts beim Beenden des Einschraubvorgangs stets in frisch verdrängtem Knochenmaterial und sind daher durch eine große Reibungskraft beaufschlagt. Das resultierende Reibungsmoment bewirkt eine sichere Arretierung der Schraube im Knochen.

In beispielhaften Ausführungsformen kann folgendes vorgesehen sein: Eine Kanülierung ermöglicht die geführte Implantation über einen Kirschnerdraht. Ein Fräskopf an der vorderen, distalen, Stirnseite ermöglicht die Implantation ohne Vorbohren. Selbstschneidende Gewinde ermöglichen die Implantation, ohne im Knochen Gewinde vorzuschneiden. Ein Antrieb an der hinteren, proximalen, Stirnseite ermöglicht eine komfortable Handhabung des Implantats. Die geeignete Wahl bestimmter Geometrieparameter verbessert die Funktion der Arretierschraube weiter.

Die oben genannten Merkmale können selbstverständlich untereinander kombiniert werden. Weitere Vorteile, Merkmale und Einzelheiten der Erfindung ergeben sich aus der nachfolgenden Beschreibung sowie anhand der Zeichnung.

Diese zeigt in:
- Fig. 1:: eine Seitenansicht einer nach dem Konzept der Erfindung ausgebildeten Arretierschraube,
- Fig. 2:: eine Längsschnittansicht der Arretierschraube gemäß Fig. 1,
- Fig. 3:: ein Detail des Außengewindes der Arretierschraube in einem ersten Axialabschnitt.
- Fig. 4:: eine Ansicht der Arretierschraube von vorne, und
- Fig. 5:: eine Ansicht der Arretierschraube von hinten.

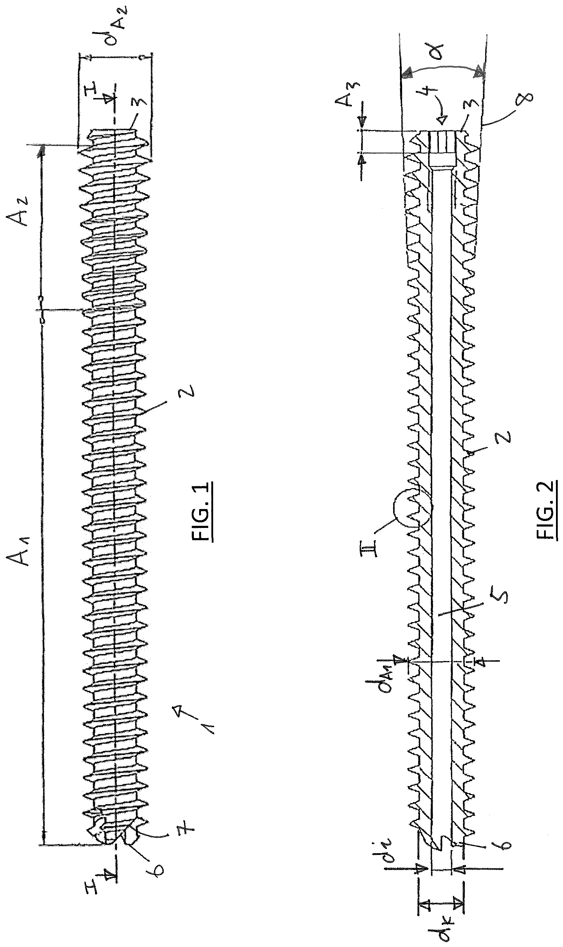

In Fig. 1 ist eine aus Metall, beispielsweise einer Titanlegierung, ausgebildete chirurgische Arretierschraube 1 zur Fixierung von Knochenstücken im Falle eines Fersenbruchs gezeigt. Die Arretierschraube 1 hat keine Kompressionsfunktion, sondern ein axial durchgehendes, hier eingängiges Außengewinde 2 mit einem axial durchgehend gleichbleibenden Kerndurchmesser d_{K}. Die Schraube kann grundsätzlich auch geeignete nichtmetalische Werkstoffe umfassen und/oder aus diesen bestehen.

Die Arretierschraube 1 kann unterteilt werden in einen ersten Axialabschnitt A₁ und einen zweiten Axialabschnitt A₂. Im ersten Axialabschnitt A₁, der durchgängig das Außengewinde 2 aufweist, verändert sich der Außendurchmesser des Außengewindes nicht. Der Außendurchmesser d_{A1} (Fig. 2) ist also konstant über die Längserstreckung des ersten Axialabschnittes A₁ und beträgt in dem gezeigten Ausführungsbeispiel 7,5 mm.

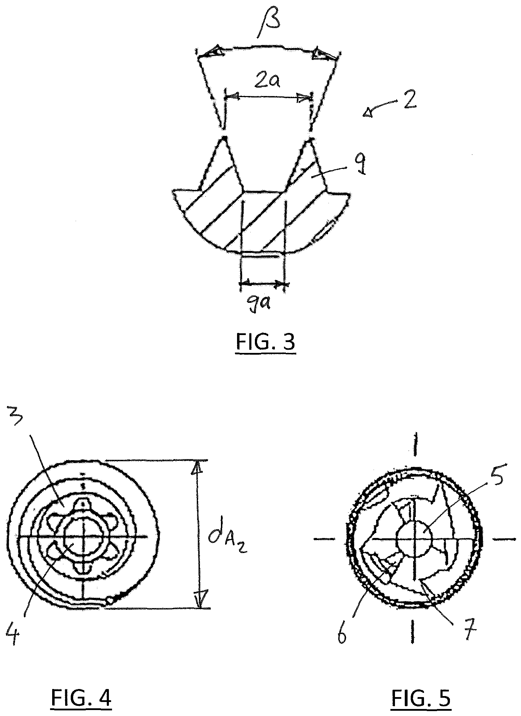

Dahingegen ist ein Außendurchmesser d_{A2} des Außengewindes 2 im zweiten Axialabschnitt A₂ nicht konstant, sondern steigt mit einer konischen Hüllkontur 8 an. Ausgehend von einem Übergang zwischen dem ersten und dem zweiten Axialabschnitt A₁, A₂, öffnet sich die konische Hüllkontur 8 mit einem Öffnungswinkel α in Richtung eines hinteren Endes 3 der Arretierschraube 1 bis zu einem maximalen Außendurchmesser d_{A2} des zweiten Axialabschnittes A₂, der gleichzeitig dem maximalen Außendurchmesser des Außengewindes 2 und dem maximalen Durchmesser der Schraube 1 insgesamt entspricht (Fig. 4). Im gezeigten Ausführungsbeispiel beträgt der Öffnungswinkel α zwischen 5° und 6° und der maximale Außendurchmesser d_{A2} 9 mm.

Dadurch, dass die Arretierschraube 1 einen axial durchgehend gleichbleibenden Kerndurchmesser d_{K} und gleichzeitig in dem zweiten Axialabschnitt A₂ einen nach hinten zunehmenden Außendurchmesser des Außengewindes 2 aufweist, wird eine sichere Positionierung der Arretierschraube 1 in der menschlichen Ferse ermöglicht, ohne dass es zu einer Kompression der Knochenteile kommt.

Das hintere, proximale, Ende 3 der Arretierschraube 1 wird von einem sich an den zweiten Axialabschnitt A₂ anschließenden, vergleichsweise kurzen Außendurchmesserabschnitt A₃ gebildet, der sich über etwa 1,5 Gewindegänge erstreckt, in denen der Außengewindedurchmesser des Außengewindes 2 nach hinten wieder abnimmt.

Fig. 3 zeigt eine vergrößerte Ansicht des Bereichs II gemäß Fig 2, in der ein Profil des Außengewindes 2 mit zwei benachbarten Gewindeabschnitten des Gewindeganges 9 erkennbar ist, der eine Gewindesteigung 2a besitzt, die über die axiale Länge der Arretierschraube 1 konstant ist. Das Außengewinde weist ferner einen Flankenwinkel β auf, der im Ausführungsbeispiel 40° beträgt. Die Gewindeabschnitte 9 sind in der Weise eines Trapezgewindes mit einem Abstand 9a voneinander beabstandet und weisen ein dreieckiges Profil auf.

Im hinteren Ende 3 der Arretierschraube 1 ist ein als Torxantrieb ausgebildeter Antrieb 4 eingebracht (Fig. 4).

Wie sich insbesondere aus Fig. 2 ergibt, ist die Arretierschraube 1 mit einer axial durchgehenden Kanülierung 5 mit einem Innendurchmesser dᵢ von in dem gezeigten Ausführungsbeispiel 2,2 mm zur Aufnahme eines Kirschnerdrahtes versehen.

Im Bereich eines vorderen, von dem Antrieb 4 abgewandten Endes 6 der Arretierschraube 1 ist diese mit Schneidmitteln 7 in der Form von Ausbuchtungen versehen, um somit eine selbstschneidende und selbstbohrende Arretierschraube zu erhalten. Die Schneidmittel 7 umfassen drei bis zum axial vorderen Ende 6 reichende Einkerbungen (Fig. 5), die dabei Windungen des Außengewindes 2 unterbrechen. Es sind drei radial verlaufende und axial wirkende Schneidkanten an der distalen Stirnseite der Schraube angeordnet. Im gezeigten Ausführungsbeispiel beträgt eine axiale Länge der Schneidmittel 7 3 mm.

Die Schneidmittel 7 ermöglichen eine Einbringung in den Knochen, ohne dass es notwendig ist, zuvor in diesen ein Gegengewinde einzuschneiden, wodurch eine optimale Gewindepassung und damit ein sicherer Halt der Arretierschraube 1 sichergestellt wird.

### Bezugszeichen

- 1: Arretierschraube
- 2: Außengewinde
- 2a: Gewindesteigung
- 3: hinteres Ende
- 4: Antrieb
- 5: Kanülierung
- 6: vorderes Ende
- 7: Schneidmittel
- 8: konische Hüllkontur
- 9: Gewindegang
- 9a: Abstand

- A₁: erster Axialabschnitt
- A₂: zweiter Axialabschnitt
- A₃: hinterer Außengewindeabschnitt

- d_{A1}: Außendurchmesser im ersten Axialabschnitt
- d_{A2}: Außendurchmesser im zweiten Axialabschnitt
- dᵢ: Innendurchmesser der Kanülierung

- d_{K}: Kerndurchmesser

## Patentansprüche

1. Chirurgische Arretierschraube zum Fixieren von Knochenteilen bei einem Fersenbruch, mit einem vorderen Ende (6), mit einem einen Antrieb (4) zum Verdrehen der Arretierschraube (1) aufweisenden hinteren Antriebsende und mit einem Außengewinde (2), welches über seine Axialerstreckung durchgehend eine gleichbleibende Gewindesteigung aufweist, wobei ein Außendurchmesser (d_{A1}) des Außengewindes (2) in einem ersten Axialabschnitt (A₁) der Arretierschraube (1) konstant ist und in einem zu dem ersten Axialabschnitt (A₁) in Richtung des hinteren Endes (3) benachbarten zweiten Axialabschnitt (A₂) mit zunehmendem Abstand zu dem vorderen Ende (6) zunimmt,
wobei ein Kerndurchmesser (d_{K}) des Außengewindes (2) im ersten und zweiten Axialabschnitt (A₁, A₂) konstant ist,
wobei das Außengewinde (2) sich axial durchgehend über die gesamte Axialerstreckung der Arretierschraube erstreckt, und
wobei sich der Kerndurchmesser (d_{K}) des Außengewindes (2) über die gesamte Axialerstreckung des Außengewindes (2) erstreckt,
**dadurch gekennzeichnet,**
**dass** axial nach hinten anschließend an den zweiten Axialabschnitt (A₂) ein, sich insbesondere über ein bis zwei Gewindegänge erstreckender, dritter Außengewindeendabschnitt (A₃) mit gegenüber dem größten Außendurchmesser (d_{A2}) des zweiten Axialabschnittes reduziertem Außengewindedurchmesser vorgesehen ist.

2. Arretierschraube nach Anspruch 1,
**dadurch gekennzeichnet,**
**dass** die Arretierschraube (1) eine axial durchgehende, zentrische Kanülierung (5) zur Aufnahme eines Kirschnerdrahtes aufweist.

3. Arretierschraube nach Anspruch 1 oder 2,
**dadurch gekennzeichnet,**
**dass** der zweite Axialabschnitt (A₂) eine konische Hüllkontur (8) aufweist.

4. Arretierschraube nach einem der vorhergehenden Ansprüche,
**dadurch gekennzeichnet,**
**dass** ein Außendurchmesserverhältnis eines maximalen Außendurchmessers (d_{A2}) des zweiten Axialabschnittes zu dem konstanten Außendurchmesser (d_{A1}) des ersten Axialabschnittes aus einem Wertebereich zwischen 1,05 und 1,4, insbesondere zwischen 1,1 und 1,3 gewählt ist und/oder 1,2 beträgt.

5. Arretierschraube nach einem der vorhergehenden Ansprüche,
**dadurch gekennzeichnet,**
**dass** ein Längenverhältnis der Gesamtlänge der Arretierschraube (1) zur Länge des zweiten Axialabschnittes (A₂) aus einem Wertebereich zwischen 2,5 und 5,0, insbesondere zwischen 3,0 und 4,5 gewählt ist.

6. Arretierschraube nach einem der vorhergehenden Ansprüche,
**dadurch gekennzeichnet,**
**dass** die Länge des zweiten Axialabschnittes (A₂) aus einem Wertebereich zwischen 10 mm und 30 mm gewählt ist und/oder 20 mm beträgt.

7. Arretierschraube nach einem der vorhergehenden Ansprüche,
**dadurch gekennzeichnet,**
**dass** die Arretierschraube (1) als selbstschneidende Schraube mit, insbesondere am vorderen Ende (6) vorgesehenen Schneidmitteln (7) ausgebildet ist.

8. Arretierschraube nach einem der vorhergehenden Ansprüche,
**dadurch gekennzeichnet,**
**dass** das Außengewinde (2) wenigstens einen mehrgängigen Abschnitt mit zwei oder mehr als zwei Gewindegängen aufweist, der sich zumindest über einen Bereich der Axialerstreckung des Außengewindes (2) erstreckt.

9. Arretierschraube nach Anspruch 8,
**dadurch gekennzeichnet,**
**dass** die mehreren Gewindegängen des mehrgängigen Abschnittes jeweils eine konstante Gewindesteigung aufweisen.

10. Arretierschraube nach einem der vorhergehenden Ansprüche,
**dadurch gekennzeichnet,**
**dass** ein maximaler Außendurchmessers (d_{A2}) des zweiten Axialabschnittes (A₂) dem maximalen Außendurchmesser des Außengewindes (2) entspricht.

11. Arretierschraube nach einem der vorhergehenden Ansprüche,
**dadurch gekennzeichnet,**
**dass** das Außengewinde (2) zumindest über einen Bereich der Axialerstreckung einen Flankenwinkel (β) umfasst, der insbesondere zwischen 35° und 45° beträgt.

12. Arretierschraube nach einem der vorhergehenden Ansprüche,
**dadurch gekennzeichnet,**
**dass** das Außengewinde (2) zumindest über einen Bereich der Axialerstreckung trapezförmig ausgebildet ist.

## Claims

1. A surgical locking screw for fixing bone parts in the case of a heel fracture, having a front end (6), having a rear drive end comprising a drive (4) for rotating the locking screw (1) and having an external thread (2) which has an invariable thread pitch over its axial extent in a throughgoing manner, wherein an outer diameter (d_{A1}) of the external thread (2) is constant in a first axial section (A₁) of the locking screw (1) and increases in a second axial section (A₂) adjacent to the first axial section (A₁) in the direction of the rear end (3) as the spacing from the front end (6) increases,
wherein a minor diameter (d_{K}) of the external thread (2) is constant in the first and second axial sections (A₁, A₂);
wherein the external thread (2) extends in an axially throughgoing manner over the total axial extent of the locking screw; and
wherein the minor diameter (d_{K}) of the external thread (2) extends over the total axial extent of the external thread (2),
**characterized in that**
a third external thread end section (A₃) which in particular extends over one to two thread turns and which has a reduced external thread diameter with respect to the largest outer diameter (d_{A2}) of the second axial section is provided axially rearwardly adjoining the second axial section (A₂).

2. A locking screw in accordance with claim 1,
**characterized in that**
the locking screw (1) has an axially throughgoing central cannulization (5) for receiving a Kirschner wire.

3. A locking screw in accordance with claim 1 or claim 2,
**characterized in that**
the second axial section (A₂) has a conical enveloping contour (8).

4. A locking screw in accordance with any one of the preceding claims,
**characterized in that**
an outer diameter ratio of a maximum outer diameter (d_{A2}) of the second axial section to the constant outer diameter (d_{A1}) of the first axial section is selected from a value range between 1.05 and 1.4, in particular between 1.1 and 1.3, and/or amounts to 1.2.

5. A locking screw in accordance with any one of the preceding claims,
**characterized in that**
a length ratio of the total length of the locking screw (1) to the length of the second axial section (A₂) is selected from a value range between 2.5 and 5.0, in particular between 3.0 and 4.5.

6. A locking screw in accordance with any one of the preceding claims,
**characterized in that**
the length of the second axial section (A₂) is selected from a value range between 10 mm and 30 mm and/or amounts to 20 mm.

7. A locking screw in accordance with any one of the preceding claims,
**characterized in that**
the locking screw (1) is configured as a self-tapping screw having cutting means (7) which are in particular arranged at the front end (6).

8. A locking screw in accordance with any one of the preceding claims,
**characterized in that**
the external thread (2) has at least one multi-turn section having two or more than two thread turns and extending at least over a region of the axial extent of the external thread (2).

9. A locking screw in accordance with claim 8,
**characterized in that**
the plurality of thread turns of the multi-turn section each have a constant thread pitch.

10. A locking screw in accordance with any one of the preceding claims,
**characterized in that**
a maximum outer diameter (d_{A2}) of the second axial section (A₂) corresponds to the maximum outer diameter of the external thread (2).

11. A locking screw in accordance with any one of the preceding claims,
**characterized in that**
the external thread (2) comprises a flank angle (β), which is in particular between 35° and 45°, at least over a region of the axial extent.

12. A locking screw in accordance with any one of the preceding claims,
**characterized in that**
the external thread (2) is configured as trapezoidal at least over a region of the axial extent.

## Revendications

1. Vis d'arrêt chirurgicale destinée à fixer des morceaux osseux en cas de fracture du talon, comportant une extrémité avant (6), une extrémité d'entraînement arrière présentant un moyen d'entraînement (4) pour faire tourner la vis d'arrêt (1), et un filetage (2) avec un pas constant en continu sur toute son extension axiale,
dans laquelle
un diamètre extérieur (d_{A1}) du filetage (2) est constant dans une première portion axiale (A₁) de la vis d'arrêt (1) et, au fur et à mesure de l'augmentation de la distance par rapport à l'extrémité avant (6), il augmente dans une seconde portion axiale (A₂) avoisinant la première portion axiale (A₁) en direction de l'extrémité arrière (3),
un diamètre de noyau (d_{K}) du filetage (2) est constant dans la première et dans la seconde portion axiale (A₁, A₂),
le filetage (2) s'étend axialement en continu sur toute l'extension axiale de la vis d'arrêt, et
le diamètre de noyau (d_{K}) du filetage (2) s'étend sur toute l'extension axiale du filetage (2),
**caractérisée en ce que**
axialement vers l'arrière, à la suite de la seconde portion axiale (A₂), il est prévu une troisième portion d'extrémité de filetage (A₃) qui s'étend sur une à deux spires de vis et qui présente un diamètre de filetage réduit par rapport au plus grand diamètre extérieur (d_{A2}) de la seconde portion axiale.

2. Vis d'arrêt selon la revendication 1,
**caractérisée en ce que**
la vis d'arrêt (1) présente un canal formant canule centrique axialement continue (5) pour recevoir une broche de Kirschner.

3. Vis d'arrêt selon la revendication 1 ou 2,
**caractérisée en ce que**
la seconde portion axiale (A₂) présente un contour enveloppe conique (8).

4. Vis d'arrêt selon l'une des revendications précédentes,
**caractérisée en ce que**
un rapport de diamètre extérieur entre un diamètre extérieur maximal (d_{A2}) de la seconde portion axiale et le diamètre extérieur constant (d_{A1}) de la première portion axiale est choisi dans une plage de valeurs comprise entre 1,05 et 1,4, en particulier entre 1,1 et 1,3 et/ou est de 1,2.

5. Vis d'arrêt selon l'une des revendications précédentes,
**caractérisée en ce que**
un rapport de longueur entre la longueur totale de la vis d'arrêt (1) et la longueur de la seconde portion axiale (A₂) est choisi dans une plage de valeurs comprise entre 2,5 et 5,0, en particulier entre 3,0 et 4,5.

6. Vis d'arrêt selon l'une des revendications précédentes,
**caractérisée en ce que**
la longueur de la seconde portion axiale (A₂) est choisie dans une plage de valeurs comprise entre 10 mm et 30 mm et/ou est de 20 mm.

7. Vis d'arrêt selon l'une des revendications précédentes,
**caractérisée en ce que**
la vis d'arrêt (1) est réalisée sous forme de vis auto-foreuse ayant des moyens de coupe (7) prévus en particulier à l'extrémité avant (6).

8. Vis d'arrêt selon l'une des revendications précédentes,
**caractérisée en ce que**
le filetage (2) comprend au moins une portion à plusieurs spires, présentant deux spires ou plus de deux spires, qui s'étend au moins sur une zone de l'extension axiale du filetage (2).

9. Vis d'arrêt selon la revendication 8,
**caractérisée en ce que**
les plusieurs spires de la portion à plusieurs spires présentent chacune un pas constant.

10. Vis d'arrêt selon l'une des revendications précédentes,
**caractérisée en ce que**
un diamètre extérieur maximal (d_{A2}) de la seconde portion axiale (A₂) correspond au diamètre extérieur maximal du filetage (2).

11. Vis d'arrêt selon l'une des revendications précédentes,
**caractérisée en ce que**
au moins sur une zone de l'extension axiale, le filetage (2) présente un angle de flanc (β) qui est compris en particulier entre 35° et 45°.

12. Vis d'arrêt selon l'une des revendications précédentes,
**caractérisée en ce que**
au moins sur une zone de l'extension axiale, le filetage (2) est réalisé en forme de trapèze.
